Europäisches Patentamt

European Patent Office

Office européen des brevets

(19)

(11) Publication number: **0 672 684 A1**

(12) **EUROPEAN PATENT APPLICATION**
**published in accordance with Art.**
**158(3) EPC**

(21) Application number: **91910163.4**

(22) Date of filing: **31.05.91**

(86) International application number:
**PCT/JP91/00739**

(87) International publication number:
**WO 91/18925 (12.12.91 91/28)**

(51) Int. Cl.⁶: **C07K 15/06**, C12P 21/02

(30) Priority: **31.05.90 JP 139809/90**

(43) Date of publication of application:
**20.09.95 Bulletin 95/38**

(84) Designated Contracting States:
**AT BE CH DE DK ES FR GB GR IT LI LU NL SE**

(71) Applicant: **MEIJA SEIKA KABUSHIKI KAISHA, LTD.**
**4-16, Kyobashi 2-chome**
**Chuo-ku, Tokyo 104 (JP)**

(72) Inventor: **MATSUNAGA, Keita, Pharmacentical Research-Center**
**Meiji Seika Kaisha, Ltd.,**
**760, Morooka-cho**
**Kohoku-ku,**
**Yokohama-shi,**
**Kanagawa 222 (JP)**
Inventor: **KURIYA, Shinichiro, Nippon Medical School**
**1-5, Sendagi 1-chome,**
**Bunkyo-ku**
**Tokyo 113 (JP)**
Inventor: **OHSAWA, Fukuichi, Pharmaceutical Research-Center**
**Meiji Seika Kaisha, Ltd.,**

**760, Morooka-cho**
**Kohoku-ku,**
**Yokohama-shi,**
**Kanagawa 222 (JP)**
Inventor: **OGATA, Kiyoyuki, Nippon Medical School**
**1-5, Sendagi 1-chome,**
**Bunkyo-ku**
**Tokyo 113 (JP)**
Inventor: **MAKABE, Osamu, Pharmaceutical Research-Center**
**Meiji Seika Kaisha, Ltd.,**
**760, Morooka-cho**
**Kohoku-ku,**
**Yokohama-shi,**
**Kanagawa 222 (JP)**
Inventor: **NOMURA, Takeo, Nippon Medical School**
**1-5, Sendagi 1-chome,**
**Bunkyo-ku**
**Tokyo 113 (JP)**

(74) Representative: **Stalla-Bourdillon, Bernard et al**
**CABINET NONY & CIE**
**29, rue Cambacérès**
**F-75008 Paris (FR)**

(54) **NOVEL MEGAKARYOCYTE COLONY STIMULATING FACTOR AND PRODUCTION THEREOF.**

(57) A homogeneous megakaryocyte colony stimulating factor (Meg-CSF) comprising a protein having an activity of forming a megakaryocyte colony from human or mouse myelloid cells in vitro and an activity of increasing the numbers of megakaryocyte precursor cells and megakaryocytes in vivo, said Meg-CSF being produced by culturing cell strains of a large human-derived pulmonary cell carcinoma, e.g. PC-13 strains, or human pulmonary carcinoma cell strains, i.e. MC-1 strains, separated as monoclonal cells from the PC-13 strains,

recovering the supernatant of the resulting culture medium, separating the Meg-CSF from the supernatant, and purifying it by a newly developed purification technique. The obtained homogeneous Meg-CSF is a homogeneous protein having a molecular weight of about 23,000 according to gel filtration or about 20,000 according to SDS-PAGE and an isoelectric point of 4.5 to 5.5. The partial amino acid sequence of the protein has been determined. The homogeneous Meg-CSF is useful for treating a certain kind of thrombocytopenia.

TECHNICAL FIELD

This invention relates to a novel megakaryocyte colony stimulating factor which is a homogeneous substance and consists of such a homogeneous protein as isolated from the supernatant of the culture obtained by cultivation of a cell strain of a human large cell lung cancer, and which factor exhibits an activity of forming magakaryocyte colonies when the factor is applied to human or murine bone marrow cells in vitro, and also which factor exhibits an activity of increasing the number of magakaryocyte progenitor cells and an activity of increasing the number of megakaryocyte in vivo. This invention also relates to a process for the preparation of said factor.

BACKGROUND ART

Platelets play important roles for promotion of thrombus formation and blood coagulation which both take place in such processes of hemostasis that the bleeding as caused by a vascular puncture can stop naturally. In humans, these platelets are released into the blood stream from the megakaryocytes which are present in the bone marrow and which have been produced by differentiation of myeloid stem cells via the megakaryocyte progenitor cells and proliferation of the latter.

Megakaryocyte colony stimulating factor (abbreviated as "Meg-CSF") is present in the blood of humans or mammals, and is a physiologically active substance which is said to act on the myeloid stem cells and/or megakaryocyte progenitor cells to promote the proliferation of these cells and the differentiation thereof into megakaryocytes. The megakaryocyte colony stimulating factor may be assayed in vitro by measuring the activity of forming the megakaryocyte colony from human or murine bone marrow cells, i.e. magakaryocyte colony stimulating activity (abbreviated as "Meg-CSA"). At present, it is found that the megakaryocyte colony stimulating activities are expressed in the urine from patients with aplastic anemia [Kawakita, M. et al., "Blood", 61, 556 (1983)], in the plasma from patients with hypomegakaryocytic thrombocytopenia [Hoffman, R. et al., "J. Clin. Invest.", 75, 1174 (1985)] and in the supernatant liquid of the cultures of human peripheral lymphocytes stimulated by phytohemagglutinin (PHA) [Messner, H.A., et al., "J. Cell Physiol. Suppl,", 1, 45]. When intention is made to recover and purify the megkaryocyte colony stimulating factor for the purpose of formulating said factor into medicinal drugs, it is necessary to obtain the urine and plasma of the above-mentioned patients as raw materials, but these urine, plasma and the like have such drawbacks that they are all biological materials, exhibit individual changes and carry potential danger of infection by virus or bacteria. For these reasons, it is difficult to obtain the raw materials for the preparation of megakaryocyte colony stimulating factor in the form of a uniform biological material in a large quantity.

On the other hand, there have been reported a megakaryocyte stimulatory factor (MSF) which has been isolated from the supernatants of the cultures of human embryonic kidney cells and which has a molecular weight of 15,000 daltons on SDS-PAGE, an isoelectric point, pI = 5.1 and the activity of promoting the synthesis of platelet factor 4 (PF4)-like protein in the megakaryocyte cells, and also a process for the preparation of MSF (see European Patent Publicaiton No. 0 260 918 A2 or Japanese patent application first publication "Kokai" No. 239298/88). This known megakaryocyte stimulatory factor (MSF) acts on megakaryocyte cells specifically but does not show megakaryocyte colony stimulating activity (Meg-CSA).

We, the present inventors, have already discovered such a cell strain of human large cell lung cancer available as a steady source for the preparation of the megakaryocyte colony stimulating factor, thus succeeded in the production of a megakaryocyte colony stimulating factor from the above-mentioned cell strain and applied for a patent application on an invention directed to obtaining a purified product of megakaryocyte colony stimulating factor (see PCT patent application No. PCT/JP89/00960 having an international filing date 1989.9.21; PCT patent application international publication WO 90/03397).

The above-mentioned PCT application discloses that the activities of the megakaryocyte colony stimulating factor have been discovered to be developed in the supernatants of the culture which are obtained by culturing a human large cell lung cancer cell strain PC-13 (commercially available from Immunobiological Laboratories, Co., Ltd., located at Fujioka City, Gumma-ken, Japan) which is available as a uniform raw material in a large quantity, that human large cell lung cancer cell strain MC-1 has been isolated from PC-13 strain by the selection of a strain having a high productivity for megakaryocyte colony stimulating factor with a view toward conducting efficient isolation of the megakaryocyte colony stimulating factor, that a cell-cultivating method for culturing the above cell strain with making use of a serum-free incubation medium has been developed, and that a purified product of the megakaryocyte colony stimulating factor has been obtained as a novel substance.

Then, we have continued our investigation and analysis on the purified product of megakaryocyte colony stimulating factor obtained earlier as above. As a result, we have now discovered that the purified product earlier obtained of megakaryocyte colony stimulating factor cannot necessarily be recognized as being completely homogeneous. Therefore, we have further proceeded with our investigation on a further purification of this factor with the intention of isolating a megakaryocyte colony stimulating factor as a purified protein which is homogeneous and practically usable as medicines, and also with the intention of utilizing megakaryocyte colony stimulating factor so isolated as the homogeneous protein in order to make such tests for determining the amino acid sequence of said protein and constructing such a DNA probe for cloning of the gene which is usable for large scale production of said factor.

Incidentally, it is known that in case where one intends to produce a physiologically active protein in a large quantity and with such a purity that said protein is homogeneous and suitable for the purpose of utilizing the protein as medicines, and if there can then be isolated and recovered such a gene which codes said protein, it is possible to utilize such genetic techniques wherein said gene is incorporated into an expression vector and then introduced into animal cell or cell of Escherichia coli so as to enable the cell to produce said protein. It is also known that if the amino acid sequence of the protein which is coded by said gene can be determined partially or entirely, it is possible to isolate and obtain the desired gene through a genetic procedure by preparing as a DNA probe such a DNA fragment having a desired DNA sequence which is deducible from the determined amino acid sequence, and utilizing said DNA probe so prepared for the isolation of said gene.

An object of this invention is to purify megakaryocyte colony stimulating factor so as to give it as a protein which is homogeneous and has such high purity that the amino acid sequence of said factor can be determined. Another object of this invention is to determine the amino acid sequence of this protein and to construct and prepare a DNA probe which is usable for the cloning of the gene.

In order to achieve these objects, we have further proceeded with our investigations with the intention of purifying the megakaryocyte colony stimulating factor earlier obtained so as to give it as a protein which is homogeneous in nature.

## DISCLOSURE OF THE INVENTION

As a result of our recent investigations as described above, we have succeeded in isolating and obtaining megakaryocyte colony stimulating factor in the form of a homogeneous protein substance as intended, by a devised new method for the purification of the megakaryocyte colony stimulating factor as detailed below. We have then found and confirmed that the homogeneous purified product of megakaryocyte colony stimulating factor now obtained by the present inventors exhibits such activities of proliferating both of the megakaryocyte progenitor cells and megakaryocyte in vivo. We have also confirmed that the homogeneous purified product of megakaryocyte colony stimulating factor now obtained is a novel substance in account of the determinations of physico-chemical properties, partial amino acid sequence and biological properties thereof. We have further found that the homogeneous purified product of megakaryocyte colony stimulating factor shows (a) a single band of molecular weight of about 20,000 when measured by SDS-PAGE, i.e. sodium dodecyl sulphate-polyacrylamide gel electrophoresis, (b) molecular weight of about 23,000 when measured by a gel-filtration method, with showing a single peak in the absorption of UV light at 280 mm, (c) a single spot when measured by two-dimensional electrophoresis and (d) a specific activity of at least $3 \times 10^7$ CFU per unit of the UV absorbance at 280 nm, whereby it is confirmed that the megakaryocyte colony stimulating factor now obtained according to this invention is a purified homogeneous substance. Further, the partial amino acid sequence of this megakaryocyte colony stimulating factor which is homogeneous has been assayed confirmatively by using automatic Edman degradation method. On the basis of these findings, this invention has been completed.

According to a first aspect of this invention, therefore, there is provided a megakaryocyte colony stimulating factor which is originated from human cells and is a homogeneous substance, characterized by having the following properties:

(a) Molecular weight:

(1) this factor has a molecular weight of about 23,000 when measured by gel-filtration method;

(2) this factor has a molecular weight of about 20,000 when measured by SDS-PAGE method in the presence of a reducing agent;

(b) Isoelectric point:

This factor shows an isoelectric point (pI) of 4.5 - 5.5 when measured by isoelectric chromatography;

(c) Specific activity:

This factor shows a specific activity of at least $3 \times 10^7$ CFU per unit of the absorbance of UV ray at 280 nm;

(d) Partial amino acid sequence:

The constitutive protein of this factor contains the following partial amino acid sequence;

Tyr - Glu - Asp - Glu - X - Pro

wherein X represents an amino acid residue not identified yet;

More particularly, the homogeneous, megakaryocyte colony stimulating factor of this invention has the following properties.

(a) Said factor can be isolated in the form of a homogeneous protein from the supernatant of the culture of human large cell lung cancer cell strain PC-13 or from the supernatant of the culture of human lung cancer cell strain MC-1 (deposited under the deposit number "FERM BP-2574" with "Fermentation Research Institute", Agency of Industrial Science and Technology, Ministry of International Trade and Industry, Government of Japan, in terms of the Budapest Treaty) having been isolated as a monoclonal strain from the PC-13 strain;

(b) Molecular weight:

(1) Said factor has molecular weight of about 23,000 when measured by gel-filtration method. A solution containing said factor alone shows a single peak each in the measured absorbance of UV ray and in the measured megakaryocyte colony stimulating activity thereof;

(2) Said factor shows a single band at the position of migration thereof corresponding to molecular weight of about 20,000 when measured by SDS-PAGE method in the presence of a reducing agent, from which said factor is recognized to have molecular weight of about 20,000 under the above condition;

(c) Isoelectric point:

This factor shows an isolectric point (pI) of 4.5 - 5.5 when measured by isoelectric chromatography;

(d) Partial amino acid sequence:

Said factor comprises the following partial amino acid sequence in the protein which constitutes said factor;

Tyr - Glu - Asp - Glu - X - Pro

wherein X represents an amino acid residue not yet identified;

(e) Thermal stability of activities:

The factor is deactivated when heated at 80°C for 60 minutes, and hence its activities are not thermally stable;

(f) Stability of activities against enzymes:

The activities of the factor remain stable when treated with neuraminidase. The factor is deactivated when treated with chymotripsin, and hence its activities are not stable against treatment with chymotripsin;

(g) Observation on the activities of cytokines and the like:

This factor is not observed to show activities of any one of interleukin-1 (IL-1), interleukin-2 (IL-2), interleukin-6 (IL-6), tumor necrosis factor (TNF) and erythropoietin (EPO).

(h) Antigenecity:

The activities of the factor are not neutralized by any one of anti-interleukin-1$\alpha$ antibody, anti-interleukin-1$\beta$ antibody, anti-human GM-CSF antibody and anti-human interleukin-3 antibody. When evaluated by radio-immunoassay, the factor does not exhibit immunological cross reaction with any one of prolactin, thyroid-stimulating hormone (TSH), growth hormone (GH), insulin, vasopressin (ADH) and adrenocorticotropic hormone (ACTH);

(i) Stability of activities against surface active agents:

When the factor is treated with sodium dodecylsulfate (SDS), the activities of the factor are partially lost and are unstable. However, the activities of the factor remain substantially stable even when the factor is treated with a non-ionic surface-active agent, such as "Tween® 20" [i.e., polyoxyethylene(20) sorbitol monolaurate], "Nonidet P40" [i.e., polyoxyethylene(9)-p-tert-octylphenol] and "Triton® X-100" [(i.e., polyoxyethylene(9-10)-p-tert-octylphenol];

(j) Characteristic physiological activities:

The factor shows the activity of forming a megakaryocyte colony, i.e. megakaryocyte colony stimulating activity, when the factor is applied to human or murine bone marrow cells in vitro. The factor also exhibits the activities of proliferating megakaryocytes and proliferating megakaryocyte progenitor cells when the factor is administered to mice;

Further, according to a second aspect of this invention, there is provided a process for the preparation of the megakaryocyte colony stimulating factor which is a homogeneous substance and has the specific properties defined in the first aspect of this invention, characterized in that the process comprises the steps of cultivating a cell strain of a human large cell lung cancer, separating from the resulting culture the supernatant containing the megakaryocyte colony stimulating factor, recovering the megakaryocyte colony stimulating factor from the supernatant and purifying said factor to give it as the protein which is in the homogeneous form.

## BEST MODE FOR WORKING THE INVENTION

In order to obtain the megakaryocyte colony stimulating factor according to this invention, generally, a cell strain of the human large cell lung cancer, especially the above-described cell strain PC-13 or human lung cancer cell strain MC-1, is cultured in a culture medium which is employed usually for the cultivation of animal cells, whereby the megakaryocyte colony stimulating factor is produced and accumulated in the supernatant of the resulting culture of the cells.

As the culture medium usable for this process, for example, RPMI-1640 medium, Dulbecco's Modified Eagle's Medium, Ham's F12 medium and the like, which are all known, can be used, either singly or in combination of two or more of them. To the culture medium may be added 0-10% of any of fetal calf serum or selenious acid, pyruvic acid, ethanolamine, transferrin, serum albumin, HEPES and the like. Preferably, the culturing of the cells can be conducted by inoculating the cells, which are to be cultured, to a culture medium, for example, at a cell concentration (initial) of $1 \times 10^4$ -$1 \times 10^6$ cells/m$\ell$, and incubating the inoculated medium at a temperature of 35-38°C at which growth and proliferation of the cells are feasible, preferably at 37°C under humidity-saturated air in the presence of 5% $CO_2$. Usable exemplary culture vessels include various flasks, Petri dishes, roller bottles and the like, which are generally employed for the culturing of animal cells. It is also possible to culture the cells in a spinner bottle by using a microcarrier. When the culturing is conducted under serum-free conditions or under conditions of a low-serum-concentration (2% or less), it is desirable to preliminarily treat a cell-adhering surface of the inner wall of each culture vessel with a cell adhesion factor such as collagen, gelatin or the like. A culturing time of 2-6 days or so is usually sufficient.

Thereafter, the supernatant of the culture is separated from the culture so obtained. This supernatant contains the megakaryocyte colony stimulating factor of this invention as produced and accumulated.

Cells of the strain PC-13 are adherent cells, which are of the type that they cannot grow and proliferate unless they are allowed to have adhered on a solid surface upon culturing of the cells. Thus, a method which is usually employed for the cloning of adherent cells may be used in order to isolate from this PC-13 strain, a monoclonal strain having a high capacity of producing megakaryocyte colony stimulating factor. For example, the limiting dilution technique, a colony-forming technique or the like can be used for that purpose. The cells, which have been obtained by any one of these techniques, are each allowed individually and separately to grow and proliferate to a cell concentration of $1 \times 10^5$ - $1 \times 10^6$ cells/m$\ell$ in a culture medium, investigating the potencies of the megakaryocyte colony stimulating factor in the supernatants of the resulting individual cultures with the aid of an "in vitro" evaluation system, thereby detecting and isolating a cloned cell strain having the high capacity to produce the megakaryocyte colony stimulating factor of this invention, and then allowing the cloned cell strain so isolated singly to grow and proliferate further, whereby the megakaryocyte colony stimulating factor can be efficiently produced.

The recovery of the megakaryocyte colony stimulating factor of this invention from the supernatant containing it therein, as well as purification of the factor can be achieved by effecting dialysis, ultra-filtration, salting-out, gel-filtration, ion-exchange chromatography, isoelectric chromatography, hydroxyapatite chromatography, hydrophobic chromatography, lectin column chromatogrphy, reversed phase chromatography, and the like, in a proper combination of two or more of them. More specifically, it is convenient to conduct the recovery and purification of the megakaryocyte colony stimulating factor, for example, by the following 6-step process:

### 1. Recovery of the active substance on hydroxyapatite column

The supernatant of the culture is caused to pass through a column of hydroxyapatite (HA) so that the active substance is adsorbed on the HA column. The active substance so adsorbed is then separated and recovered from the column by elution. Desirably, this elution may be effected with an aqueous solution of sodium phosphate or potassium phosphate, whose pH and concentration are approximately 7 - 8 and 0.2 - 0.5 M, respectively. The elution may be conducted while monitoring the absorbance of the eluate fractions for ultraviolet ray (UV) of 280 nm wavelength which the proteins absorb. Eluate fractions showing a UV absorption at this wave-length value are combined together and collected as an active fraction containing the active substance.

2. Purification by chromatography on Con A-conjugated carrier column

The active fraction as eluted and collected above is caused to pass, as it is, through a column of Con A (Concanavalin A)-conjugated carrier, for example, a Con A-conjugated agarose column, and fractions having passed without being adsorbed in this column are collected. Among the fractions so collected, such active fractions showing the UV absorption at 280 nm are combined together in a similar manner to the above-described procedure by monitoring the UV absorbance at 280 nm. The active fractions thus combined are called the "Con A-nonbound fraction".

3. Purification by hydrophobic chromatography

To the Con A-nonbound fraction above-mentioned, ammonium sulfate was added in an amount of 30 - 45% saturation under stirring, and after the lapse of 1 - 12 hours the mixture was centrifuged to give a supernatant. The supernatant was passed through a hydrophobic carrier column to adsorb the active substance on the carrier column which was then eluted to recover the adsorbed fractions. As illustrative hydrophobic carriers, there may be used those having such hydrophobic radical which is suitable for the separation of protein, typically TSK gel Butyl-TOYOPEARL 650 (product of TOSOH CORP.), TSK gel Phenyl-TOYOPEARL 650 (product of TOSOH CORP.), Butyl-Sepharose (product of Pharmacia Co.), Phenyl Sepharose (product of Pharmacia Co.), Phenyl-Superose (product of Pharmacia Co.) and the like. The adsorption is desirably effected by using a buffer solution containing ammonium sulfate in an amount of 30 - 45% saturation at pH 7 - 9 and at a concentration of about 10 - 50 mM, and the elution is desirably effected by using a buffer solution containing ammonium sulfate in an amount of 0 - 45% saturation at pH 7 - 9 and at a concentration of about 10 - 50 mM in the manner of concentration gradient technique with ammonium sulfate.

4. Purification by hydroxyapatite chromatography

The active fractions obtained in the preceding hydrophobic chromatography are adsorbed on a hydroxyapatite (HA) column and then recovered by elution. Desirably, the adsorption is carried out by using a sodium or potassium phosphate buffer solution at pH 7 - 9 and at a concentration of about 0.01 - 0.05 M and the elution is effected by using a sodium or potassium phosphate buffer solution in a concentration gradient manner at pH 7 - 9 and at a concentration of about 0.01 - 0.5 M.

5. Purificaion by anion-exchange chromatography

The active fractions obtained in the preceding hydroxyapatite chromatography are desalted by dialysis, ultra-filtration or the like or diluted to lower the salt concentration and then passed through a colomn of an anion exchanger to adsorb the active substance thereon. The active substance so adsorbed is then desorbed by elution from the anion-exchanger and recovered as active fractions. Illustrative anion-exchangers include DEAE-TOYOPEARL (product of TOSOH CORP.), QAE-TOYOPEARL (product of TOSOH CORP.), DEAE-Sepharose (product of Pharmacia Co.), Q-Sepharose (product of Pharmacia Co.), Mono-Q (product of Pharmacia Co.) and the like. Desirably, the adsorption of the active substance on the anion-exchanger is effected at pH 7 - 9 and at a salt concentration of about 0.01 - 0.05 M and the elution is effected by salt concentration gradient method in the range of about 0.1 - 1 M and at pH 7 - 9.

6. Purification by cation-exchange chromatography

The active fractions obtained in the preceding anion-exchange chromatography are desalted by dialysis, ultra-filtration or the like or diluted to lower the salt concentration and then passed through a column of a cation-exchanger to adsorb the active substance thereon. The active substance so adsorbed is then desorbed by elution from the cation-exchanger and recovered as active fractions. Illustrative cation-exchangers include CM-TOYOPEARL (product of TOSOH CORP.), SP-TOYOPEARL (product of TOSOH CORP.), CM-Sepharose (product of Pharmacia Co.), S-Sepharose (product of Pharmacia Co.), Mono-S (product of Pharmacia Co.), IEC SP-420N (product of Showa Denko K.K.) and the like. Desirably, the adsorption of the active substance on the cation-exchanger is carried out at pH 4 - 6 and at a salt concentration of about 0.01 - 0.05 M and the elution is carried out by salt concentration gradient method in the range of about 0.1 - 1 M at pH 4 - 6. The active substance intended is taken up from appropriate active fractions chosen from among the eluted fractions thus recovered. Thus, the appropriate active fractions which contain the desired megakaryocyte colony stimulating factor by itself are collected as a solution of the aimed active substance. If necessary, this solution may be further desalted by dialysis to afford an aqueous solution of the desired megakaryocyte colony stimulating factor in a homogeneous purified form.

Detection of the megakaryocyte colony stimulating factor of this invention in each of the purification steps above can be effected by using, as a marker, the megakaryocyte colony stimulating activity (Meg-CSA) in vitro.

By the way, the process for recovery and purification comprising the above-mentioned six steps is given merely to illustrate the recovery of a purified product of the megakaryocyte colony stimulating factor of this invention. Of course, the recovery and purification may be achieved by means of other procedures.

The homogeneous, megakaryocyte colony stimulating factor according to this invention promotes the processes of "in vitro" formation of the megakaryocyte colony from human or murine bone marrow cells, that is, it has a megakaryocyte colony stimulating activity. Further, this factor also accelerates "in vivo" proliferation of megakaryocyte progenitor cells and megakaryocytes. The megakaryocyte colony stimulating factor is useful as a reagent for studying the differentiation from megakaryocyte progenitor cells to megakaryocyte cells and the proliferation of megakaryocyte cells, and also be useful as medicinal products.

The megakaryocyte colony stimulating factor according to this invention can be effectively employed for therapeutic treatment of certain thrombocytopenia, namely, thrombocytopenia after administration of anti-cancer drugs, thrombocytopenia involved by radio-theraphy, thrombocytopenia due to megakaryocyte colony stimulating factor deficiency, thrombocytopenia due to aplastic anemia, and thrombocytopenia occurring after bone marrow transplantation. Further, the megakaryocyte colony stimulating factor of this invention can also be useful for therapeutic treatment of leukemia by differentiating megakaryoblastic leukemia cells to megakaryocytes. In addition, the megakaryocyte colony stimulating factor of this invention can also be employed as a substitute or auxiliary agent for platelets upon transfusion of the latter. The homogeneous, megakaryocyte colony stimulating factor of this invention may be administered intraperitoneally, intravenously or subcutaneously, and may be mixed with various conventional excipients to formulate medicinal compositions of various forms which can be administered.

Further, the homogeneous purified product of megakaryocyte colony stimulating factor of the present invention may be utilized to determine partial amino acid sequence of this substance, whereby a DNA probe can be prepared for cloning of the gene.

BRIEF DESCRIPTION OF THE DRAWINGS

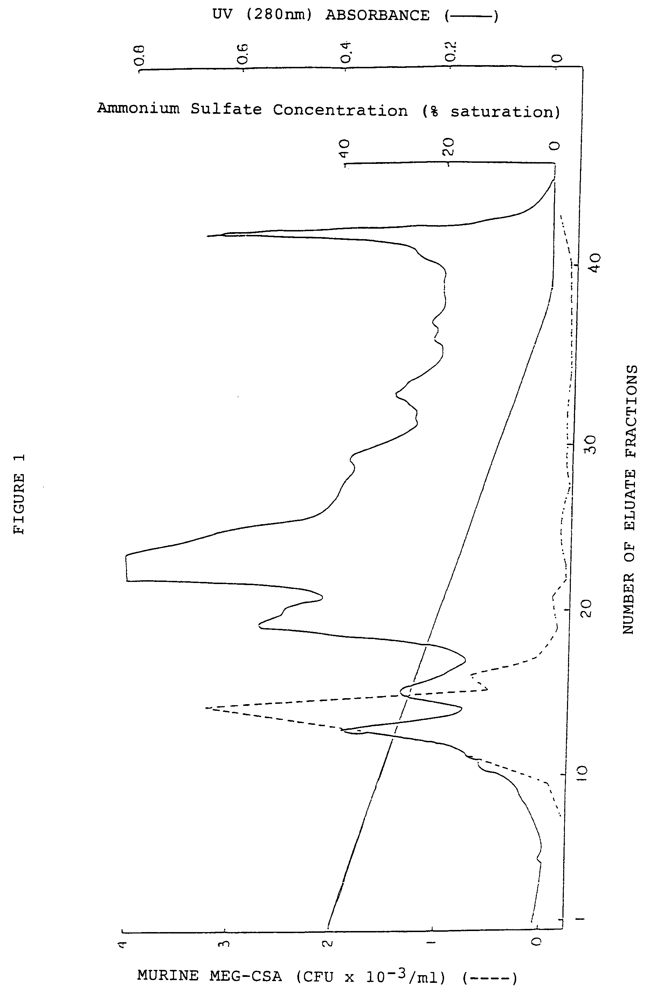

FIGURE 1 shows a chromatogram of representing the pattern that the megakaryocyte colony stimulating activities (shown by a broken line) of the eluate fractions and the absorbance (shown by a solid line) of the eluate fractions for ultraviolet ray (UV) at 280 nm wavelength are changed in relation to the order of elution of the respective eluate fractions, the said eluate fractions having been collected in the course of further purification of the megakaryocyte colony stimulating factor which had been partially purified in the preceding steps, and said further purification being effected by hydrophobic chromatography with a hydrophobic carrier, TSK gel Phenyl-Toyopearl 650 (a product of TOSOH Corp.) in the process of Example 4 given later which demonstrates an illustrative production of the megakaryocyte colony stimulating factor of this invention in the form of a homogeneous, purified product;

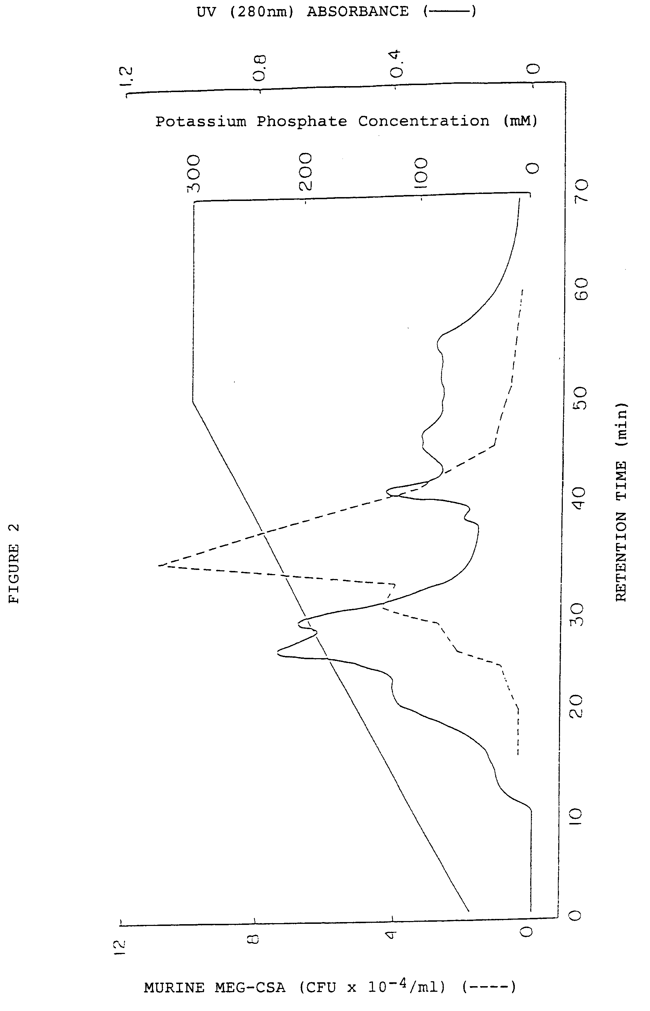

FIGURE 2 shows a chromatogram for depicting the pattern that the megakaryocyte colony stimulating activities (shown by a broken line) of the eluate fractions and the absorbance (shown by a solid line) of the eluate fractions for ultraviolet ray (UV) at 280 nm wavelength are changed in relation to the retention time of the respective eluate fractions, the said eluate fractions having been collected in the course of further purification of the megakaryocyte colony stimulating factor which had been partially purified by the hydrophobic chromatography in Example 4 given later, and said further purification being effected by passing the said partially purified factor through a hydroxyapatite column (a product of Kanto Chemical Co., Inc.) as adsorber;

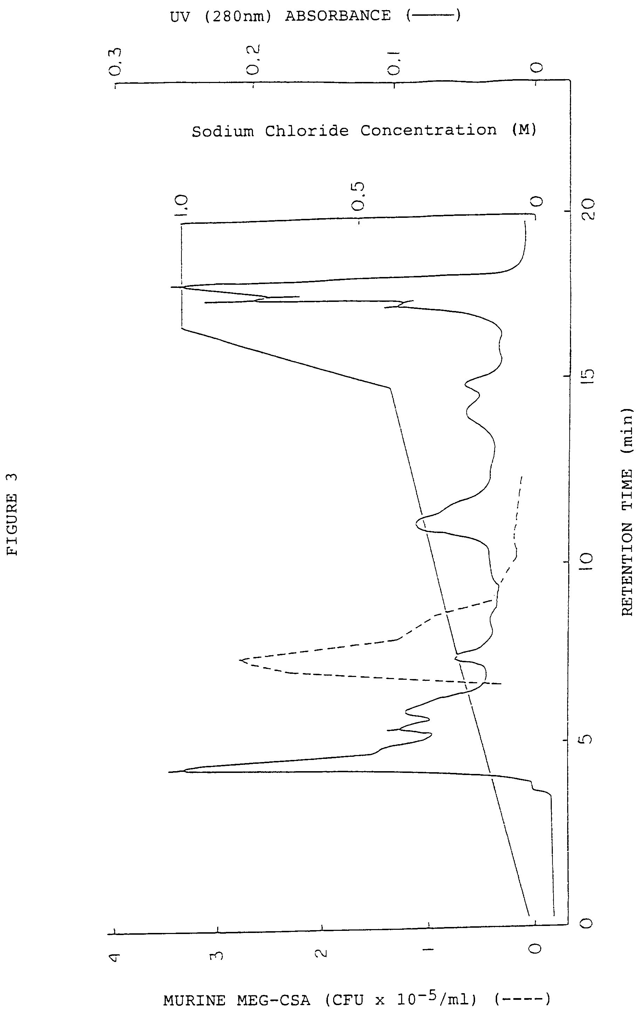

FIGURE 3 shows a chromatogram for depicting the pattern that the megakaryocyte colony stimulating activities (shown by a broken line) of the eluate fractions and the absorbance (shown by a solid line) of the eluate fractions for ultraviolet ray (UV) at 280 nm wavelength are changed in relation to the retention time of the respective eluate fractions, the said eluate fractions having been collected in the course of further purification of the megakaryocyte colony stimulating factor which had been purified in the purification step with the hydroxyapatite column in Example 4 given later, and said further purification being effected by anion-exchange chromatography with an ion-exchanger, mono Q (product of Pharmacia Company);

8

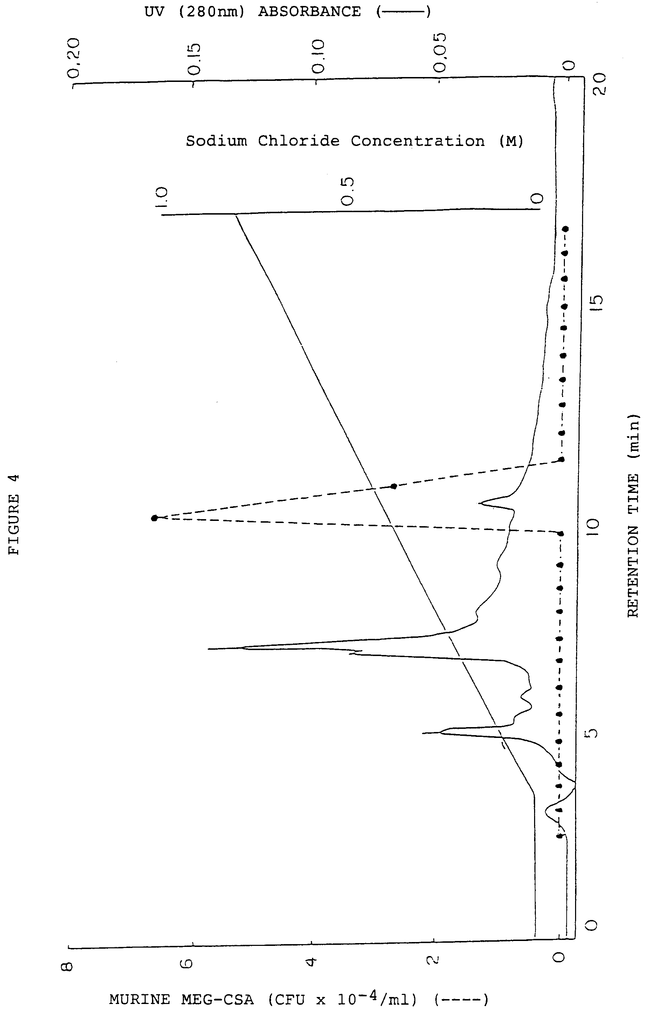

FIGURE 4 shows a chromatogram for representing the pattern that the megakaryocyte colony stimulating activities (shown by a broken line) of the eluate fractions and the absorbance (shown by a solid line) of the eluate fractions for ultraviolet ray (UV) at 280 nm wavelength are changed in relation to the retention time of the respective eluate fractions, the said eluate fractions having been collected in the course of further purification of the megakaryocyte colony stimulating factor which had been purified in the purification step with the Mono Q column in Example 4 given later, and said further purification being effected by cation-exchange chromatography with a cation-exchanger, IEC SP-420N (product of Showa Denko K.K.); and

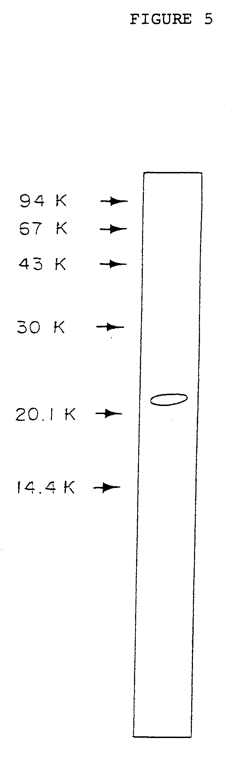

FIGURE 5 shows a diagram of electrophoresis given by analyzing the megakaryocyte colony stimulating factor obtained as the final, purified product in Example 4 given later, with the analysis being made by sodium dodecyl sulfate-polyacrylamide gel electrophoresis (SDS-PAGE) in the presence of a reducing agent. The diagram shows a single band of this factor. In Figure 5, the arrows show the positions of migration of the proteins used as the standard and the numbers given at the arrows each show the molecular weights (kilodalton) of the proteins used as the standard.

Example 1

The activities of the megakaryocyte colony stimulating factor which was present in the supernatant of the culture as obtained by culturing a cell strain of a human large cell lung cancer in accordance with this invention, were investigated by the following two methods, namely human plasma clot method and murine fibrin clot method.

(1) Human plasma clot method

An aqueous solution of calcium chloride (40 $\mu\ell$, 2.6 $\mu$g/m$\ell$) was added to 360 $\mu\ell$ of Iscove's modified Dulbecco's medium (hereinafter abbreviated as "IMDM") which contained 20 $\mu\ell$ of bovine plasma, 40 $\mu\ell$ of human Group-AB plasma, 4 mg of bovine serum albumin, 10 $\mu$M of 2-mercaptoethanol, 2 x $10^5$ human bone marrow mononuclear cells and 40 $\mu\ell$ of a sample with or without the megakaryocyte colony stimulating factor to be assayed. The resulting mixture was immediately placed at the center of a 35-mm petri dish so that the mixture was allowed to coagulate. IMDM (0.6 m$\ell$) was placed around the clot as formed. The samples to be assayed were respectively the supernatant as recovered from the culture of human large cell lung cancer cell strain PC-13, the RPMI-1640 medium employed for the culturing of the cell strain PC-13 (as a blank control), and as a positive control, the supernatant of the culture of phytohemagglutinin-stimulated human peripheral lymphocytes (PHA-LCM) which are known to have the human megakaryocyte colony stimulating activities. The petri dishes each containing the respective clots above were incubated at 37°C for 12 days in the presence of air containing 5% of $CO_2$. While identifying the megakaryocyte colonies as formed in the clots with the anti-human factor VIII antibody (product of Dakopatts Co.), the human megakaryocyte colony stimulating activities (Meg-CSA) were assayed. The results are shown in Table 1 below. The supernatant of the culture as obtained by culturing the cells of PC-13 strain was found to exhibit the human megakaryocyte colony stimulating activities similarly to the PHA-LCM.

Table 1

| Sample | Human Meg-CSA (CFU/m$\ell$) |
|---|---|
| RPMI-1640 medium | 0.5 |
| Supernatant from the culture of cells of PC-13 strain | 43 |
| PHA-LCM | 125 |

(2) Murine fibrin clot method

A mixture of 80 $\mu\ell$ of fetal calf serum, 0.2 mg of bovine fibrinogen (product of Sigma Co.), 0.2 units of bovine thrombin (product of Sigma Co.), 2 x $10^5$ murine bone marrow cells of $BDF_1$ mouse (male), and 400 $\mu\ell$ of IMDM containing 40 $\mu\ell$ of a sample with or without the megakaryocyte colony stimulating factor to be assayed, imediately after said mixture was prepared, was placed at the center of a 35-mm petri dish so that the mixture was allowed to coagulate. Iscove's modified Dulbecco's medium (IMDM, 0.6 m$\ell$) was

9

placed around the clot as formed. Samples to be assayed were respectively the supernatant of the culture as obtained by culturing human large cell lung cancer cell strain PC-13, the RPMI-1604 medium employed for the culturing of the cells of PC-13 strain (as a blank control), and, as a positive control, the supernatant of the culture of murine myelomonocytic leukemia cell (WEHI-CM) which is known to have the murine megakaryocyte colony stimulating activities. The petri dishes each containing the respective clots above were incubated at 37°C for 6 days in the presence of air containing 5% of $CO_2$. The murine megakaryocyte colony stimulating activities were assayed while identifying the megakaryocyte colonies as formed in the clots by the acetylcholinesterase staining. The results are shown in Table 2 below. The supernatant of the culture as obtained by culturing the cells of PC-13 strain was found to exhibit the murine megakaryocyte colony stimulating activities similarly to the WEHI-CM.

Table 2

| Sample | Murine Meg-CSA (CFU/mℓ) |
|---|---|
| RPMI-1640 medium | 1 ± 1 |
| Supernatant from the culture of cells of PC-13 strain | 36 ± 4 |
| WEHI-CM | 49 ± 9 |

Example 2

After the cells of PC-13 strain in the logarithmic growth phase were suspended at an adjusted concentration of 2 cells/mℓ in an RPMI-1640 medium containing 10% of fetal calf serum, the resultant cell suspension was placed in the wells of a 96-well microplate at a rate of 0.1 mℓ/well, followed by incubation at 37°C in the presence of 5% $CO_2$.

Fifty-seven monoclonal cell strains, which had been obtained by the incubation of the cells in the individual wells, were separately removed and then allowed to proliferate. The proliferated cells of the 57 separate strains were individually inoculated at the inoculum size of 1 x $10^5$ cells/mℓ in 20-mℓ aliquots of an RPMI-1640 medium containing 5% of fetal calf serum, followed by incubation at 37°C for 4 days in the presence of 5% $CO_2$.

The resulting supernatants of the cultures were separately dialyzed against Dulbecco's PBS of a 1/20-fold concentration for the desalting and then lyophilized. Crude powder samples containing the megakaryocyte colony stimulating factor thus obtained were individually dissolved in 1 mℓ aliquots of distilled water. The murine megakaryocyte colony stimulating activity of each of the resulting solutions was evaluated by the murine fibrin clot method above-mentioned. The results were such that the aqueous solution of the lyophilized supernatant of the culture of PC-13 strain obtained as above exhibited the activity of 270 CFU/mℓ and that in contrast therewith the aqueous solution of the lyophilized supernatant of the culture of one monoclonal strain having a higher ability to produce the megakaryocyte colony stimulating factor than that of PC-13 strain exhibited the activity of 470 CFU/mℓ. This monoclonal strain was thus deemed to have been obtained as a clonal strain of high productivity and designated as "human lung large cell cancer cell strain MC-1".

This human lung large cell cancer cell strain MC-1 has been deposited under the deposit number of "FERM BP-2574" in a depository "Fermentation Research Institute", Tsukuba City, Ibaragi Prefecture, Japan, in accordance with the provisions of the Budapest treaty.

Example 3

Cells of MC-1 strain above-mentioned which belong to the cells of a human lung large cell cancer were cultured at a concentration of 1 x $10^5$ cells/mℓ and at 37°C for 4 days in the presence of 5% $CO_2$ in a serum-free medium having the composition indicated below in Table 4 (namely, RPMI-HPTS medium as described above). Thereafter, 100 mℓ of the culture supernatant were recovered from the resulting culture. The supernatant thus obtained was used as samples to be assayed and was dialyzed against Dulbecco's PBS of a 1/20-fold concentration for desalting, followed by lyophilization of the dialyzate. The resulting dried powder containing the megakaryocyte colony stimulating factor was dissolved in 5 mℓ of distilled water. The murine megakaryocyte colony stimulating activities of the resultant aqueous solution were evaluated by the murine fibrin clot method. The results are shown in Table 3 below. As a control was employed such powder as obtained by processing RPMI-HPTS medium in the same manner as described above, except that the inoculation with cells of MC-1 strain was omitted.

As a result, it became clear that the cells of MC-1 strain produce the megakaryocyte colony stimulating factor even when they are cultured in a serum-free medium.

Table 3

| Sample | Murine Meg-CSA (CFU/mℓ) |
|---|---|
| Supernatant from the culture of MC-1 strain | 640 ± 70 |
| RPMI-HPTS medium (control) | 17 ± 10 |

Table 4

Composition of RPMI-HPTS medium (per 10ℓ)

| | |
|---|---|
| RPMI-1640 medium, as a powder form (product of Nissui Pharmaceutical Co., Ltd.) | 104 g |
| Sodium hydrogen carbonate (product of Nacalai Tesque, Inc.) | 12 g |
| Penicillin G (product of Meiji Seika Kaisha, Ltd.) | 400 mg |
| Streptomycin (product of Meiji Seika Kaisha, Ltd.) | 400 mg |
| Transferrin (product of Boelinger GmbH) | 50 mg |
| Selenious acid (product of Nacalai Tesque, Inc.) | 200 μg |
| Sodium pyruvate (product of Sigma Co., Ltd.) | 1.0 mM |
| HEPES (product of Nacalai Tesque, Inc.) | 15.0 mM |

Example 4

This example illustrates a method which is suitable for culturing the human lung large cell cancer cell strain MC-1, namely a cell strain having a high productivity of megakaryocyte colony stimulating factor, and recovering the megakaryocyte colony stimulating factor from the resultant supernatants of the resultant cultures, followed by purifying it.

(1) Culturing

MC-1 strain (FERM BP-2574) as obtained in Example 2 was allowed to proliferate at 37°C under humidity-saturated air in the presence of 5% of $CO_2$ in an RPMI-1640 medium containing 5% of fetal calf serum (product of Nissui Pharmaceutical Co., Ltd.) in petri dishes until $1 \times 10^9$ cells were obtained. Those cells of MC-1 strain were washed with Bulbecco's PBS, followed by treatment with a trypsin-EDTA solution (Product of GIBCO Co.). Two to five minutes later, the enzyme reaction was terminated by a trypsin inhibitor (product of Sigma Co., Ltd.). Cells which had been removed and collected from the wall of the culture vessel by pipetting were washed with Dulbecco's PBS and then suspended in a serum-free medium to be used. After the number of the cells was counted, they were used as the inoculating cells.

The serum-free medium employed here for the production of megakaryocyte colony stimulating factor was the abve-described RPMI-HPTS medium which contained RPMI-1640 medium as a basal medium.

As the culture vessel, were used tissue-culturing, square-shaped petri dishes (25 x 25 cm in sizes, a product of Nunc Company) whose inner wall had been treated with collagen ("CELL MATRIX-1P"; product of Iwaki Co., Ltd.). The MC-1 strain cells were inoculated, at a rate of $1 \times 10^7$ cells per petri dish, to 100 petri dishes in each of which 125 mℓ of RMPI-HPTS medium had been placed and incubated beforehand at 37°C under humidity-saturated air in the presence of 5% $CO_2$. The cells in those dishes were cultured at 37°C under humidity-saturated air in the presence of 5% $CO_2$. In the course of the culturing, the culture medium was replaced by aliquots of the fresh medium eight times at intervals of 3 - 4 days, whereby totally 100 liters of the supernatants of the cultures were recovered.

(2) Recovery of the active substance from the culture supernatant

The resultant supernatant was filtered. The filtrate was forced to pass at a rate of 25 liters per column at the flow rate of 60 mℓ/min through columns of hydroxyapatite (90 mm in diameter x 60 mm in length; products of Seikagaku Kogyo Co., Ltd.) which had been equilibrated with a 10 mM potassium phosphate buffer (pH 7.5), so that the active substance was adsorbed on the hydroxyapatite. After each column was washed with 3,200 mℓ of a 10 mM potassium phosphate buffer (pH 7.5) at a flow rate of 80 mℓ/min, the active substance was eluted with a 0.5 M potassium phosphate buffer (pH 7.5) at a flow rate of 3.5 mℓ/min.

(3) Chromatography on Con A-Agarose column

The eluate from the preceding step was directly passed at a flow rate of 3.5 mℓ/min. through a Con A-agarose column (22 mm in diameter x 310 mm in length, product of Seikagaku Kogyo Co., Ltd.) which had been equilibrated with 10 mM potassium phosphate buffer (pH 7.5), and the effluents from the column were collected in 35-mℓ fractions. Monitoring was made by measuring the ultraviolet ray (280 mm) absorbance of each of said fractions which passed through the column, so that the fractions exhibiting the UV absorbance were collected as active fractions. The absorbance unit of the ultraviolet ray (280 nm) of the active fractions collected was 2375. After desalting by dialysis, the active fractions totally had the Meg-CSA of $50 \times 10^5$ CFU.

(4) Hydrophobic chromatography

To a 1250 mℓ portion of the active fractions which had passed through the Con A-agarose column above, ammonium sulfate was added in an amount to give a 40% saturation, and the resulting mixture was stirred for one hour and then centrifuged to give a supernatant. The supernatent thus obtained was passed, at a flow rate of 8 mℓ/min, through Phenyl-TOYOPEARL 650S column (50 mm in diameter and 82 mm in length; product of TOSOH Corp.) which had been equilibrated with 20 mM Tris-hydrochloric acid buffer (pH 7.5) containing ammonium sulfate at 40% saturation, so that the active substance was adsorbed on the column. After washing the column with the same buffer solution as above, the active substance was eluted from the column with 20 mM Tris-hydrochloric acid buffer (pH 7.5) containing ammonium sulfate at 40 - 0%

12

saturation in a gradient elution manner, at a flow rate of 4 mℓ/min. The eluate was collected in 40 mℓ-fractions and each of the fractions was assayed by measurements of ultraviolet ray (at 280 mm) absorbance and of murine megakaryocyte colony stimulating activities. Figure 1 of the accompanying drawings shows changes in the UV absorbance and in the megakaryocyte colony stimulating activities which occurred with the elution sequences of each fraction. The changes in the UV absorbance are indicated by the solid curve, while the changes in the activities are shown by the broken curve. This equally applies to Figure 2 and Figure 3 of the accompanying drawings. On the other hand, in the gradient elution process above, the active fraction which was eluted at the ammonium sulfate concentration of about 25% saturation was taken up as the required active fraction. The absorbance unit of UV (at 280 nm) in this required active fraction was 87 (total Meg-CSA : 12.1 x $10^5$ CFU).

(5) Chromatography on hydroxyapatite column

A 240 mℓ portion of the active fraction as recovered from the column of the preceding hydrophobic chromatography was dialyzed against a 50 mM potassium phosphate buffer (pH 7.2) containing 50 mM sodium chloride. The resulting dialyzate was passed, at a flow rate of 1 mℓ/min, through a hydroxyapatite column (8 mm in diameter and 100 mm in length; product of Kanto Chemical Co.) which had been equilibrated with 50 mM potassium phosphate buffer (pH 7.2), so that the active substance was adsorbed on the hydroxyapatite (HA). The column was then eluted with 50 mM-300 mM potassium phosphate buffers (pH 7.2) in a gradient elution manner, at a flow rate of 1 mℓ/min. The eluate was collected in 2 mℓ-fractions. The ultraviolet ray (280 nm) absorbance and murine megakaryocyte colony stimulating activities of each fraction were measured. The results of measurement are shown in Figure 2 of the accompanying drawings in term of changes in those measured values as brought about with the lapse of retention time of each fraction. Active fraction which was eluted at the phosphate concentration of about 225 mM was collected. The absorbance unit of UV (280 nm) of this active fraction was 2.3 (total Meg-CSA : 7.9 x $10^5$ CFU).

(6) Anion exchange chromatography

A 6 mℓ portion of the active fraction as recovered from the hydroxyapatite column was dialyzed against a 5 mM Tris-hydrochloric acid buffer (pH 7.5) + 5 mM sodium chloride. The resulting dialyzate was added, at a flow rate of 1 mℓ/min, to a column of an ion-exchanger, Mono Q HR5/5 (5 mm in diameter and 50 mm in length; product of Pharmacia Company) which had been equilibrated with the same buffer as above, so that the active substance was adsorbed on the Mono Q column. After the column was washed with the same buffer, the active substance was eluted with a 5 mM Tris-hydrochloric acid buffer (pH 7.5) containing 5mM - 400 mM sodium chloride in a gradient elution manner, at a flow rate of 1 mℓ/min. The eluate was collected in 500 μℓ fractions. The ultraviolet ray (at 280 nm) absorbance and murine megakaryocyte colony stimulating activities of each fraction were measured. Figure 3 of the accompanying drawings shows changes in the UV absorbance and megakaryocyte colony stimulating activities of each fraction as brought about with the lapse of retention time of the fractions. Active fraction which was eluted at the NaCℓ concentraiton of about 200 mM was collected. The absorbance unit of UV (280 nm) of this active fraction was 0.13 (total Meg-CSA : 3.4 x $10^5$ CFU).

(7) Cation exchange chromatography

To a 0.45 mℓ portion of the active fraction as recovered from the preceding Mone Q column was added a 10 mM MES [2-(N-Morpholino)ethanesulfonic acid]-NaOH buffer (pH 5.0) + 5mM sodium chloride (4.05 mℓ), and the resulting solution was added, at a flow rate of 0.22 mℓ/min, to a column of an ion-exchanger, IEC SP-420N (4.6 mm in diameter and 35 mm in length; product of Showa Denko K.K.) which had been equilibrated with the same buffer as above, so that the active substance was adsorbed on the IEC SP-420N column. After the column was washed with the same buffer as abve, the active substance was eluted with a 10 mM MES-NaOH buffer (pH 5.0) containing 5mM - 1,000 mM sodium chloride in a gradient elution manner, at a flow rate of 0.85mℓ/min.

The eluate was collected in 500 μℓ fractions and the ultraviolet ray (280 mm) absorbance and megakaryocyte colony stimulating activities were measured for each of the fractions collected. Figure 4 of the accompnying drawings shows changes in the UV absorbance and in the megakaryocyte colony stimulating activities of each fraction as brought about with the lapse of retention time of the fractions. Active fraction as eluted at the NaCℓ concentration of about 400 mM was taken up. The absorbance unit of ultraviolet ray (280 mm) of this active fraction was 0.0018 (total Meg-CSA : 5.5 x 10$^4$ CFU).

The active fraction thus recovered was dialyzed against a 10 mM sodium phosphate buffer (pH 7.2) + 150 mM sodium chloride for the desalting, whereby there was obtained an aqueous solution containing a megakaryocyte colony stimulating factor which had a specific activity of 3.0 x 10$^7$ CFU per absorbance unit of ultraviolet ray (280 nm) as a water-soluble, final purified product.

Table 5 given below summarily shows values of absorbance unit of UV (280 nm) and of total Meg-CSA-(CFU) for the active fractions recovered in the individual steps for the recovery and purification as above-mentioned.

Table 5

| Active fractions obtained in individual steps for recovery and purification | Meg-CSA (CFU) | Absorbance of UV(280 mm) (Absorbance unit) |
|---|---|---|
| Active fraction recovered from the culture supernatant through HA column | - | - |
| Con A-agarose | 50 x 10$^5$ | 2375 |
| Hydrophobic chromatography | 12.1 x 10$^5$ | 87 |
| HA chromatography | 7.9 x 10$^5$ | 2.3 |
| Mono Q chromatography | 3.4 x 10$^5$ | 0.13 |
| SP-420N chromatography | 5.5 x 10$^4$ | 0.0018 |

Example 5

Properties of the megakaryocyte colony stimulating factor of this invention, which was obtained as the final purified product in the last step of Example 4, were investigated as follows:

(1) Molecular weight

① The molecular weight of the megakaryocyte colony stimulating factor which was obtained as the final purified product above (hereinafter refered to as "the present substance") was measured by electrophoresis with sodium dodecyl sulfatepolyacrylamide gel (SDS-PAGE) in the presence of a reducing agent. Figure 5 of the accompanying drawings shows a diagram of the electrophoresis. The calculation of the molecular weight of "the present substance" in SDS-PAGE method was made by comparison with phosphorylase b (molecular weight : 94,000), bovine serum alubumin (molecular weight : 67,000), ovalbumin (molecular weight : 43,000), carbonic anhydrase (molecular weight : 30,000), soybean-trypsin inhibitor (molecular weight : 20,100) and α-lactalbumin (molecular weight : 14,400) used as standard proteins having known values of molecular weight. As a result, "the present substance" showed a single band in the presence of a reducing agent and was found to have a molecular weight of about 20,000.

Further, "the present substance", when analyzed by two-dimensional electrophoresis (O'Farrell method), was detected as a single spot which positioned at pI of 4.5 - 5.5 and at molecular weight of 20,000. This clearly shows that "the present substance" is a homogeneous substance.

② The molecular weight of "the present substance" under the unmodified conditions was measured by a gel-filtration method using a high-performance liquid chromatography carried out under the conditions as mentioned below. As a result, "the present substance" was eluted at retention time of 24 - 25 minutes which correspond to the molecular weight of about 23,000. The eluate fraction thus collected was found to exhibit a single absorption peak of UV (280 mm) and a single activity peak. Thus, the molecular weight of "the present substance" as measured by a gel-filtration method was about 23,000.

Column : TSK gel G 2000 SW$_{XL}$ (7.8 mm in diameter and 300 mm in length, product of TOSOH Corp.)

Solvent : 10 mM Tris-hydrochloric acid buffer (pH 7.5) + 150 mH NaCl

Flow rate : 0.5 mℓ/min.

The calculation of the molecular weight of "the present substance" in the above gel-filtration method was made by comparison with bovine serum albumin (molecular weight: 67,000), ovalbumin (molecular weight: 43,000), carbonic anhydrase (molecular weight: 29,000) and ribonuclease A (molecular weight: 13,700) as the standard proteins having known values of the molecular weight.

(2) Isoelectric point

Using isoelectric chromatography, the isoelectric point of "the present substance" was measured under the conditions mentioned below. Thus, "the present substance" was eluted at such point that the eluate has pH values of 4.5 - 5.5 and the eluate fractions so collected exhibited a single UV (280 nm) absorption peak and a single activity peak. As a result, "the present substance" was found to have the isoelectric point of pI = 4.5 - 5.5.

| | |
|---|---|
| Column : | "Mono P" (product of Pharmacia Company) |
| Initiator solvent : | 25 mM bis-Tris-iminodiacetate buffer (pH 7.1) |
| Developer solvent : | 10% polybuffer 74-iminodiacetate buffer (pH 4.0) |
| Flow rate : | 0.5 mℓ/min. |

(3) Partial amino acid sequence

"The present substance" was digested with lysyl endopeptidase and the resulting digest was separated by reverse-phase chromatography. The peptide thus obtained was analyzed by automatic Edman degradation method using a vapor phase protein sequencer (manufactured by Applied Biosystems, Model 447A) to determine the amino acid sequence of the peptide. As a result, the protein which constitute "the present substance" were found to contain the following partial amino acid sequence:

Tyr - Glu - Asp - Glu - X - Pro

wherein X is an amino acid residue not yet identified.

The partial amino acid sequence of the formula above is one which does never exist in any of human proteins already known. Therefore, it has now been confirmed that "the present substance" is a novel substance which is different from any known human proteins.

(4) Thermal stability

Samples of "the present substance" were held at 37°C, 56°C and 80°C, respectively, for 60 minutes in Dulbecco's PBS (product of Nissui Pharmaceutical Co., Ltd.), and the residual murine megakaryocyte colony stimulating activities were measured. The results are shown in Table 6. "The present substance" was unstable at 80°C.

Table 6

| Temperature | 37°C | 56°C | 80°C |
|---|---|---|---|
| Residual activities (%) | 78 | 13 | 0 |

(5) Stability to enzyme

"The present substance" was treated at 37°C for 5 hours with 0.5 unit of insoluble neuraminidase (product of Sigma Chemical Co., Ltd.) in a 0.1 M aqueous sodium acetate solution (pH 5.0). The residual murine megakaryocyte colony stimulating activities were evaluated. "The present substance" was stable to neuraminidase. "The present substance" was treated, on the other hand, with 10 μg/mℓ of chymotrypsin in Dulbecco's PBS (product of Nissui Pharmaceutical Co., Ltd.) at 37°C for 4 hours and the residual murine megakarycyte colony stimulating activities were evaluated. The results of these tests are shown in Table 7. "The present substance" was unstable to chymotrypsin.

Table 7

| Enzyme | Residual activities (%) |
|---|---|
| Non-addition | 100 |
| Neuraminidase | 100 |
| Chymotrypsin | 21 |

(6) Activities of cytokines

Investigation was made as to whether "the present substance" has the respective activities of known cytokines, namely, IL-1, IL-2, IL-6, TNF and EPO. The methods of measuring these respective activities were as follows:

[IL-1]

IL-1-sensitive human melanoma cells, namely cell strain A375, were cultured and the resulting cells were then incubated for 4 days in the presence of "the present substance" or IL-1 as a standard sample, after which the degree of inhibiting the proliferation of the cells was determined by vital staining method with Neutral Red. The potency of IL-1 was calculated through its comparison with that obtained by using the standard sample.

[IL-2]

Murine IL-2 dependent cells, namely the cell strain CTLL-2, were cultured. The resulting cells were incubated overnight in the presence of "the present substance" or IL-2 as a standard sample, followed by pulse-labelling with [$^3$H]thymidine. The potency of IL-2 was calculated through its comparison with that obtained by using the standard sample.

[IL-6]

SKW6-C1 strain originated from the B-cells were cultured. The resulting cells were incubated for 4 days in the presence of "the present substance" or IL-6 as a stadard sample. Induction of IgM production was measured by ELISA. The potency of IL-6 was calculated through its comparison with that obtained by using the standard sample.

[TNF]

Murine fibroblasts, L929 strain, was incubated by the monolayer culture method. The resulting cells were further incubated overnight in the presence of "the present substance" or TNF as a standard sample, followed by pulse-labelling with [$^3$H]thymidine. The cytotoxicity occurred on the L929 strain cells was determined through its comparison with that obtained by using the standard sample.

[EPO]

Murine fetal liver cells were incubated for 20 hours in the presence of "the present substance" or EPO as a standard sample. The medium was then replaced, followed by pulse-labelling with [$^{54}$Fe]iron citrate. The potency of EPO was determined through its comparison with that obtained by using the standard sample.

The results of respective deteminations are shown in Table 8.

Table 8

| Activities | Potency value as measured |
|---|---|
| IL-1 | Less than the detectable limit |
| IL-6 | ditto |
| IL-6 | ditto |
| TNF | ditto |
| EPO | ditto |

(7) Antigenecity

Investigation was made as to whether "the present substance" has immunological reactivity with known cytokines and hormones.

Firstly, there were carried out experiments wherein "the present substance" was reacted with an anti-human IL-1 antibody for neutralization purpose. Rabbit polyclonal antibodies (product of Genzyme Corp.) for human IL-1$\alpha$ and human IL-1$\beta$ were used as antibodies, and the level of each neutralizing antibody in the neutralization reaction mixture was adjusted to 200 NU/m$\ell$. As a result, the megakaryocyte colony stimulating activities of "the present substance" were not reduced even when it was treated with anti-human IL-1$\alpha$ antibody or anti-human IL-1$\beta$ antibody. It was hence found that "the present substance" does not immunologically react with the antibodies described above.

Similarly, experiments for neutralization of "the present substance" were conducted by reaction with anti-human GM-CSF antibody and anti-human IL-3 antibody. Rabbit polyclonal antibodies (product of Genzyme Corp.) for human GM-CSF and human IL-3 were used as antibodies. As a result, the activities of "the present substance" were not reduced even when it was treated with anti-human GM-CSF antibody or anti-human IL-3 antibody. It was thus found that "the present substance" does not immunologically react with the antibodies described above. "The present substance" was further investigated by radio-immunoassay, when "the present substance" was not detected by antibodies against prolactin, thyroid-stimulating hormone (TSH), growth hormone (GH), insulin, vasopressin (ADH) or adrenocorticotropic hormone (ACTH). It was accordingly found that "the present substance" is immunologically distinct from these hormones.

(8) Stability to surface active agents

Investigation was made as to whether "the present substance" remains stable when it was treated with surface active agents. Thus, "the present substance" was placed in Dulbecco's PBS and then incubated at 4°C for 24 hours in the presence of various surface active agents. After the surface active agents were removed by dialysis from the resultant incubated solutions, the murine megakaryocyte colony stimulating activities still remaining in the solutions were determined. As a result, "the present substance" was partially deactivated and was unstable when it was treated with a 2% aqueous SDS solution. On the other hand, "the present substance" remained stable when it was treated with a non-ionic surface-active agent, such as 0.02% Tween 20®, 0.01% Nonidet NP-40 and 0.01% Triton® X-100 in solution in water. The results of these stability experiments are summarized in Table 9.

Table 9

| Surface active agent | Residual activities (%) |
|---|---|
| Control | 100 |
| SDS | 26 |
| Tween® 20 | 86 |
| Nonidet NP-40 | 90 |
| Triton® X-100 | 103 |

Example 6

"In vivo" effects of the homogeneous, megakaryocyte colony stimulating factor of this invention, which had been obtained as the final purified product in Example 4, on megakaryocyte cells were investigated.

(1) Effects on murine megakaryocyte progenitor cells

Each of several 0.2 m$\ell$ samples of PBS (phosphate buffered saline), which contained the megakaryocyte colony stimulating factor of this invention at concentrations of 0, 40, 80 and 160 CFU, respectively, was intraperitoneally administered once to BDF$_1$ mice (male, 8-weeks old). Upon an elapsed time of 48 hours after the administration, the mice were sacrificed. The numbers of megakaryocyte progenitor cells (CFU-Meg) in the excised spleen were counted. The counting of megakaryocyte progenitor cells was conducted by a murine fibrin clot method where the spleen cells were incubated with addition of a 5% proportion of a cell suspension of the spleen cells in 4 m$\ell$ of IMDM and also with addition of a 5% proportion of the supernatant of the culture of poke weed-mitogen-stimulated spleen cells (PWM-SCM). The number of acetylcholinesterase-staining positive cells which had been matured by PWM-SCM was counted. The number of megakaryocyte progenitor cells (CFU-Meg) which were contained in the spleen was calculated so as to evaluate the dependency of the CFU-Meg proliferating activity of "the present

17

substance" on the dosage. The results as obtained are shown in Table 10.

Table 10

| Dosage of "the present substance" | Number of CFU-Meg/spleen |
|---|---|
| 0 CFU | 4200 ± 800 (100%) |
| 40 CFU | 5600 ± 1000 (133%) |
| 80 CFU | 6900 ± 1300 (64%) |
| 160 CFU | 8500 ± 900 (202%) |

(2) Effects on megakaryocytes in the mouse spleen

PBS containing the homogeneous, megakaryocyte colony stimulating factor of this invention at a concentration of 400 CFU/0.20 ml was intraperitoneally administered once to C3H/Hej mice (male, 8-weeks old). Upon elapsed times of 0, 24, 48 and 72 hours after the administration, the mice were sacrificed successively. The number of megakaryocytes in the spleen as excised from each mouse was counted immediately. Upon counting of the cells, the spleen was fixated with aqueous 10% formaldehyde and embedded in paraffin. A sectioned specimen of the spleen was then prepared from the spleen to give the largest cross-sectional area in the specimen: After the sectioned specimen was subjected to HE staining (hematoxylineosin staining), the number of megakaryocytes so stained was counted under a microscope and the number of megakaryocytes per unit area of the red pulp was then calculated. The results obtained are summarized in the Table 11.

Table 11

| Time elapsed after administration | Number of megakaryocytes per $mm^2$ of red pulp | Level of significance |
|---|---|---|
| 0 hr. | 7.2 ± 2.2 (100%) | |
| 24 hrs. | 10.4 ± 0.6 (144%) | $p < 0.05$ |
| 48 hrs. | 8.7 ± 2.5 (120%) | |
| 72 hrs. | 13.0 ± 3.9 (179%) | $p < 0.05$ |

INDUSTRIAL APPLICABILITY

As has been described above, this invention has made it feasible to culture a cell strain of a human large cell lung cancer and then to isolate, from the supernatant of the resultant cell culture, a homogenous purified product of a megakaryocyte colony stimulating factor (Meg-CSF) having the above-described physiological activities. The homogeneous, megakaryocyte colony stimulating factor is utilizable as a medicine.

**Claims**

1.  A megakaryocyte colony stimulating factor which is originated from human cells and is a homogeneous substance, characterized by having the following properties:
    (a) Molecular weight:
        (1) this factor has a molecular weight of about 23,000 when measured by gel-filtration method;
        (2) this factor has a molecular weight of about 20,000 when measured by SDS-PAGE method in the presence of a reducing agent;
    (b) Isoelectric point:
    This factor shows an isoelectric point (pI) of 4.5 - 5.5 when measured by isoelectric chromatography;
    (c) Specific activity:
    This factor shows a specific activity of at least $3 \times 10^7$ CFU per unit of the absorbance of UV ray at 280 nm;

(d) Partial amino acid sequence:

The constitutive protein of this factor contains the following partial amino acid sequence;

Tyr - Glu - Asp - Glu - X - Pro

wherein X represents an amino acid residue not identified yet.

2. A process for the preparation of a megakaryocyte colony stimulating factor having the properties as specified in Claim 1, characterized in that the process comprises the steps of cultivating a cell strain of a human large cell lung cancer, separating from the resulting culture the supernatant containing the megakaryocyte colony stimulating factor, recovering the megakaryocyte colony stimulating factor from the resulting supernatant and purifying said factor to give it as the protein which is in the homogeneous form.

3. A process for the preparation of a megakaryocyte colony stimulating factor as claimed in Claim 2 wherein the cell strain of the human large cell lung cancer is human large cell lung cancer cell strain PC-13 or human large cell lung cancer cell strain MC-1.

FIGURE 1

FIGURE 2

FIGURE 3

EP 0 672 684 A1

FIGURE 4

FIGURE 5

# INTERNATIONAL SEARCH REPORT

International Application No PCT/JP91/00739

## I. CLASSIFICATION OF SUBJECT MATTER (if several classification symbols apply, indicate all) 6

According to International Patent Classification (IPC) or to both National Classification and IPC

Int. Cl⁵  C07K15/04, C12P21/02//A61K37/02
(C12P21/02, C12R1:91)

## II. FIELDS SEARCHED

Minimum Documentation Searched 7

| Classification System | Classification Symbols |
|---|---|
| IPC | C07K15/04, C12P21/00, 21/02 |

Documentation Searched other than Minimum Documentation
to the Extent that such Documents are Included in the Fields Searched 8

Biological Abstracts Data Base (BIOSIS)

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| X | WO, A1, 90/3397 (Meiji Seika Kaisha, Ltd.), April 5, 1990 (05. 04. 90) | 1-3 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| August 20, 1991 (20. 08. 91) | September 9, 1991 (09. 09. 91) |
| International Searching Authority | Signature of Authorized Officer |
| Japanese Patent Office | |

Form PCT/ISA/210 (second sheet) (January 1985)